## Europäisches Patentamt

## European Patent Office

## Office européen des brevets

(19)

(11) Veröffentlichungsnummer: **0 014 867**
**B1**

(12)

# EUROPÄISCHE PATENTSCHRIFT

(45) Veröffentlichungstag der Patentschrift:
06.01.82

(51) Int. Cl.³: **C 07 C 99/02**

(21) Anmeldenummer: 80100459.9

(22) Anmeldetag: 30.01.80

(54) Verfahren zur Gewinnung von Leucin aus Proteinhydrolysaten.

(30) Priorität: 16.02.79 DE 2906034

(43) Veröffentlichungstag der Anmeldung:
03.09.80 Patentblatt 80/18

(45) Bekanntmachung des Hinweises auf die Patenterteilung:
06.01.82 Patentblatt 82/1

(84) Benannte Vertragsstaaten:
BE FR GB NL

(56) Entgegenhaltungen:
FR-A-853 173
FR-A-856 378
FR-A-1 080 525
FR-A-2 409 981
GB-A-1 314 708
US-A-2 929 840
CHEMICAL ABSTRACTS, Band 56, Nr. 11,
28. Mai 1962, Spalte 13007 g,
Columbus, Ohio, US

CHEMICAL ABSTRACTS, Band 56, Nr. 11,
28. Mai 1962, Spalte 13007 h,
Columbus, Ohio, US

(73) Patentinhaber: SOCIETE DES PRODUITS NESTLE S.A.,
Case postale 353, CH-1800 Vevey (CH)

(72) Erfinder: Steinmetzer, Walter, im Bodetal 9,
D-3334 Süpplingen (DE)

CHEMICAL ABSTRACTS, Band 63, Nr. 3,
2. August 1965, Spalte 3197,
Columbus, Ohio, US,
S.SHESTAKOV et al.: »The complex production
of amino acids from technical raw material
containing keratin«

J. P. GREENSTEIN AND M. WINITZ:
»Chemistry of the Amino Acids«,
Band 3, John Wiley & Sons,
New York, US,
Seite 1888−9 »Illustrative procedure« 24−2;
Seite 2052−4
»Isolation from protein«; Seite 2085;
»Illustrative procedure« 31-1;
Seite 2351 »Illustrative procedure« 40−3

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Verfahren zur Gewinnung von Leucin aus Proteinhydrolysaten

Die vorliegende Erfindung betrifft ein Verfahren zur Gewinnung von Leucin aus Proteinhydrolysaten, bei welchem nach Neutralisation der Hydrolysate eine fraktionierte Filtration zu verschiedenen Zeiten durchgeführt wird.

Für die Gewinnung von L-Aminosäuren werden verschiedene pflanzliche oder tierische Proteine längere Zeit mit Salzsäure oder Schwefelsäure erhitzt und aus den Hydrolysaten die gewünschten Aminosäuren auf verschiedene Weise isoliert.

Für die Gewinnung bestimmter Aminosäuren werden oft Rohstoffe mit optimaler Aminosäurezusammensetzung verwendet. Bekannt ist die Verwendung von keratinhaltigen Rohstoffen wie Haare, Federn oder Borsten für die Gewinnung von Cystin, die Verwendung von Hämoglobin oder Maiskleber für die Herstellung von L-Leucin, die Verwendung von Kasseinhydrolysaten für die Isolierung von L-Tyrosin oder die Verwendung von Eialbumin für die Herstellung von L-Phenylalanin (S. Greenstein and Winitz, »Chemistry of the amino acids«).

Der Begriff »optimale Aminosäurezusammensetzung« bezieht sich auf den Gehalt an einer bestimmten Amionosäure, welche aus dem Protein isoliert werden soll. So hat z. B. das Kasein einen sehr hohen Tyrosingehalt von 8,1% und das Eialbumin hat wiederum einen sehr hohen Phenylalaningehalt von 7,7%. Ein optimales Protein für die Gewinnung von L-Leucin ist das Zein im Maiskleber. Dieses Protein hat einen Leucingehalt von 23,7%, während nur 0,9% Cystin vorhanden sind.

Diese ausgewählten Rohstoffe enthalten die zu isolierende Aminosäure in hohen Konzentrationen, und der Gehalt an störenden Begleit-Aminosäuren ist gering. So kann aus diesen Spezialhydrolysaten die gewünschte Aminosäure sehr oft auf eine relativ einfache Methode ausgefällt werden. Nach FR-A-2 409 981 wird das Hydrolysat von Haaren neutralisiert und das ausgefällte Rohcystin mit geringen Verunreinigungen abfiltriert. Nach FR-A-856 379 wird aus einem Hydrolysat von Maiskleber bei pH 6 ein Gemisch von Leucin und Tyrosin ausgefällt. Aus dieser Mischung wird dann bei einem pH von 2,4 erneut Tyrosin gefällt und das in Lösung verbliebene Leucin durch Eindampfen der Mutterlauge von der Tyrosinfällung gewonnen. Nach FR-A-853 173 wird aus einem Maiskleberhydrolysat Tyrosin bei pH 2,4 und Leucin bei pH 0,9 bis 1,0 ausgefällt und nach dreimaliger Umkristallisation Leucin mit 90% Reinheit gewonnen.

Nach den US-A-2 929 840 wird aus einem Hydrolysat von Weizenkleber durch Neutralisation eine Mischung von Cystin, Tyrosin und Leucin ausgefällt. Aus dieser Mischung wird dann bei pH 2,4 Cystin und Tyrosin gefällt, während das Leucin in Lösung bleibt.

Nachteile dieser Methoden sind die Abhängigkeit von bestimmten ausgewählten Rohstoffen bei einer Gewinnung der Aminosäuren, und außerdem ist die Reinheit der so gewonnenen L-Aminosäuren für bestimmte Verwendungszwecke nicht ausreichend. Bei der heutigen umfangreichen Verwendung von L-Aminosäuren in Infusionslösungen für die parenterale Ernährung werden sehr hohe Anforderungen an die Qualität gestellt. So müssen diese Aminosäuren eine Reinheit von 99% aufweisen.

Ziel der vorliegenden Erfindung ist eine Gewinnung von L-Leucin sehr hoher Reinheit aus Proteinhydrolysaten verschiedenster Herkunft, welche Gewinnung einfacher ist und eine gute Ausbeute mit weniger Aufwand ermöglicht, d. h. industriell verwendbar ist.

Zu diesem Zweck ist das erfindungsgemäße Verfahren zur Gewinnung von Leucin aus Proteinhydrolysaten, bei welchem nach Neutralisation der Hydrolysate eine fraktionierte Filtration zu verschiedenen Zeiten durchgeführt wird, dadurch gekennzeichnet, daß durch die fraktionierte Filtration eine an Cystin und Tyrosin und eine an Leucin angereicherte Fraktion erhalten, die leucinreiche Fraktion in Säure gelöst, der Gehalt an Isoleucin in der Lösung auf 1,0 – 1,5% eingestellt, durch Zusatz von Oxydationsmitteln Methionin oxydiert, ein Rohleucin bei einem in Abhängigkeit des Tyrosingehaltes eingestellten pH-Wert von 1,0 bis 2,0 ausgefällt und durch Wiederholung des Fällungsvorganges im gleichen pH-Bereich das Leucin weiter gereinigt wird.

So kann das zur Durchführung des vorliegenden Verfahrens zu verwendende Ausgangsmaterial aus den verschiedensten Proteinquellen wie z. B. Oeltrester, Mikroorganismen, insbesondere Hefen oder Getreidekeim erhalten werden.

Bei der Isolierung von L-Leucin aus normalen Proteinhydrolysaten stört u. a. die Anwesenheit von Cystein und Tyrosin, da diese beiden Aminosäuren am schwersten löslich sind und auch bei pH-Werten von 0,9 – 1,0 gemeinsam mit dem Leucin auskristallisieren. Es wurde nun gefunden, daß während der Durchführung einer an sich bekannten fraktionierten Filtration nach Neutralisation der Hydrolysate die schwerlöslichen Aminosäuren mit unterschiedlichen Geschwindigkeiten auskristallisieren.

Die erste Fraktion, welche unmittelbar nach der Neutralisation ausfällt, enthält die Aminosäuren Leucin, Isoleucin, Phenylalanin, Methionin und Valin neben geringen Mengen an Tyrosin. Die zweite Fraktion, welche erst nach einer längeren Kristallisationsdauer ausfällt, ist an Tyrosin und Cystin angereichert. Durch diese Vortrennung der Aminosäuren in zwei Gruppen können Hydrolysate verschiedenster Herkunft für die Gewinnung von Leucin verwendet werden. Dies bedeutet eine größere Freiheit bei der Auswahl der Rohstoffe für die Aminosäuregewinnung.

Die erste Fraktion, die weiter oben sowie nachfolgend die leucinreiche Fraktion genannt wird, enthält also die Aminosäuren Leucin, Isoleucin, Methionin, Phenylanin und Valin mit geringen Anteilen an Tyrosin. Die beiden isomeren Aminosäuren Leucin und Isoleucin weisen sehr ähnliche physikalische Eigenschaften auf und sind deshalb schwer zu trennen.

Es wurde gefunden, daß beim Übergang von der dipolaren zur Kationenform in einem ganz bestimmten pH-Bereich die Löslichkeit von Isoleucin gegenüber Leucin rascher ansteigt. In einer gesättigten Kochsalzlösung sind bei einem pH-Wert von 6,0 0,4% Leucin und 0,5% Isoleucin gelöst. Bei einem pH-Wert von 1,5 sind 1,1% Leucin und 1,7% Isoleucin gelöst. Durch Einstellen der Lösung auf eine definierte Isoleucin-Konzentration kann im pH-Bereich von 1,0 bis 2,0 Leuicin ausgefällt und von Isoleucin getrennt werden. Zusätzliche Schwierigkeiten ergeben sich bei Anwesenheit von Methionin, da diese Aminosäure in allen pH-Bereichen mit Leucin Copräzipitate bildet. Deshalb wird das Methionin vor der Fällung des Leucins durch Oxydation in lösliches Methioninsulfoxyd überführt.

Was den Gehalt an Isoleucin in der Lösung anbelangt, so kann dieser durch Zusatz von Wasser auf 1,0 bis 1,5% eingestellt werden.

Der genaue pH-Wert im Bereich von 1,0 bis 2,0 wird als Funktion des Tyrosingehaltes gewählt. Bei höheren Tyrosingehalten wird der pH-Wert erniedrigt, und bei niedrigeren Tyrosingehalten wird der pH-Wert erhöht. Nach Abtrennung des Leucins wird dieser Fällungsvorgang im gleichen pH-Bereich wiederholt und das Leucin weiter gereinigt.

## Beispiel

Nach Neutralisation eines sauren Proteinhydrolysates werden die ausgefällten schwer löslichen Aminosäuren als leucinreiche erste Fraktion abgetrennt. Diese Aminosäuremischung wird bei einem pH-Wert von 0,5 gelöst und durch Zusatz von Wasser die Isoleucinkonzentration auf ca 1,5% eingestellt. Nach Ermittlung des Methioningehaltes wird durch Zusatz der äquimolaren Menge Wasserstoffperoxyd das Methionin bei 90°C oxidiert. Anschließend wird die Lösung nach Zusatz von Aktivkohle filtriert, der pH-Wert mit verdünnter Natronlauge auf 1,0 bis 1,5 eingestellt und nach dem Abkühlen das auskristallisierte Rohleucin abgetrennt. Das Rohleucin wird erneut bei pH 0,5 gelöst und durch Fällung bei 1,0 bis 2,0 weiter gereinigt. Dieses Verfahren wird so oft wiederholt, bis die gewünschte Reinheit des L-Leucins erreicht ist.

Die Auswahl des pH-Wertes im Bereich 1,0 bis 2,0 richtet sich nach dem Tyrosingehalt in der Lösung. Für 0,65; 0,8; 1,0 und 1,2% Tyrosin in der Lösung werden die pH-Werte von 1,8; 1,65; 1,52 und 1,42 gewählt.

## Patentanspruch

Verfahren zur Gewinnung von Leucin aus Proteinhydrolysaten, bei welchem nach Neutralisation der Hydrolysate eine fraktionierte Filtration zu verschiedenen Zeiten durchgeführt wird, dadurch gekennzeichnet, daß durch die fraktionierte Filtration eine an Cystein und Tyrosion und eine an Leucin angereicherte Fraktion erhalten, die leucinreiche Fraktion in Säure gelöst, der Gehalt an Isoleucin in der Lösung auf 1,0 – 1,5% eingestellt, durch Zusatz von Oxydationsmitteln Methionin oxydiert, ein Rohleucin bei einem in Abhängigkeit des Tyrosingehaltes eingestellten pH-Wert von 1,0 bis 2,0 ausgefällt und durch Wiederholung des Fällungsvorganges im gleichen pH-Bereich das Leucin weiter gereinigt wird.

## Claim

A process for recovering leucine from protein hydrolysates, in which a fractional filtration is carried out at different times after neutralisation of the hydrolysates, characterised in that a tyrosine- and cystine-rich fraction as well as a leucine-rich fraction are obtained by the fractional filtration, the leucine-rich fraction is dissolved in acid, the isoleucine content of the solution is adjusted to between 1.0 and 1.5%, the methionine is oxidised by the addition of oxidising agents, a crude leucine is precipitated at a pH-value adjusted to between 1.0 and 2.0 in dependence upon the tyrosine content, and the leucine is further purified by repeating the precipitation process in the same pH-range.

## Revendication

Procédé d'obtention d'acides aminés à partir d'hydrolysats de protéines, dans lequel on réalise une filtration fractionnée dans différents délais après neutralisation des hydrolysats, caractérisé par le fait que l'on obtient par la filtration fractionnée une fraction riche en cystine et en tyrosine et une fraction riche en leucine, on dissout la fraction riche en leucine dans l'acide, on ajuste la teneur en isoleucine de la solution à 1,0 – 1,5%, on oxyde la méthionine par addition d'un moyen d'oxydation, on obtient une leucine brute par précipitation à un pH ajusté entre 1,0 et 2,0 en fonction de la teneur en tyrosine, et l'on purifie la leucine par répétition du processus de précipitation dans le même domaine de pH.